Europäisches Patentamt

European Patent Office

Office européen des brevets

(11) Numéro de publication: **0 287 469 B1**

# FASCICULE DE BREVET EUROPEEN

(45) Date de publication de fascicule du brevet: **08.07.92**

(51) Int. Cl.⁵: **A61K 35/78**, A61K 31/485,
//(A61K35/78,31:485),
(A61K31/485,31:35)

(21) Numéro de dépôt: **88400902.8**

(22) Date de dépôt: **14.04.88**

(54) Assocation renfermant à titre de principes actifs, un analgésique central et la forskoline.

(30) Priorité: **15.04.87 FR 8705362**

(43) Date de publication de la demande:
**19.10.88 Bulletin 88/42**

(45) Mention de la délivrance du brevet:
**08.07.92 Bulletin 92/28**

(84) Etats contractants désignés:
**CH DE GB IT LI NL**

(56) Documents cités:
**EP-A- 0 192 056**
**WO-A-85/02616**
**GB-A- 1 550 410**
**GB-A- 1 589 326**

(73) Titulaire: **ROUSSEL-UCLAF**
**35, Boulevard des Invalides**
**F-75007 Paris(FR)**

(72) Inventeur: **Delevallée, Françoise**
**48-50, Avenue de la Dame Blanche**
**F-94120 Fontenay Sous Bois(FR)**
Inventeur: **Potier, Pierre**
**14, Avenue de Breteuil**
**F-75007 Paris(FR)**

(74) Mandataire: **Bourgouin, André et al**
**Département des Brevets ROUSSEL UCLAF**
**111, route de Noisy B.P. no 9**
**F-93230 Romainville(FR)**

## Description

L'invention a pour objet l'association renfermant, à titre de principes actifs :
- a) un analgésique central choisi parmi la morphine, la péthidine, le fentanyl, la pentazocine et le dextromoramide,
- b) la forskoline, pour une utilisation simultanée, séparée ou étalée dans le temps, pour une méthode de traitement analgésique du corps humain ou animal.

La forskoline est un dérivé de type labdane dont la structure est donnée par exemple dans le brevet allemand DE-A-2.557.784.

De nombreuses propriétés pharmacologiques ont été décrites pour ce produit : propriétés anti-hypertensives, antiagrégantes plaquettaires, bronchodilatatrices, stimulatrices de l'adénylate cyclase /Drugs of the future 4,26 (1979) et 7 (1) 55 (1982)/ anti-inflammatoire et oxygénatrice (brevet allemand DE-A-3.502.686).

Par ailleurs, les analgésiques centraux ou narcotiques sont considérés classiquement comme possédant les mêmes caractéristiques que la morphine ; ils provoquent une toxicomanie et entrainent une dépression respiratoire.

Les dérivés de synthèse de la morphine les plus utilisés en thérapeutique sont la péthidine, le destromoramide et la pentazocine. Les dérivés présentent les mêmes inconvénients que la morphine et la recherche de dérivés possédant le même puissant effet analgésique que la morphine et dépourvus d'action toxicomanogène est restée jusqu'à présent infructueuse.

Or, il vient d'être trouvé que si l'on associe la forskoline avec un analgésique central, on aboutit à un effet analgésique supérieur à celui auquel on aurait pu s'attendre avec l'analgésique seul.

C'est ainsi qu'au cours d'une expérimentation chez l'animal, la potentialisation de l'effet d'un analgésique a été obtenue en l'associant à une dose inactive de forskoline.

L'association, objet de l'invention, permet donc d'obtenir une action analgésique importante, tout en employant une quantité d'analgésique central inférieure à celle qui serait nécessaire pour obtenir la même activité et de diminuer ainsi les effets secondaires de l'analgésique utilisé.

On voit évidemment l'intérêt de l'association, objet de l'invention, en thérapeutique humaine, puisqu'elle permet de réduire les effets indésirables des différents analgésiques centraux employés en thérapeutique.

L'analgésique central utilisé dans l'association est de préférence la morphine ou un dérivé de synthèse succédané de la morphine.

Les effets indésirables liés à l'administration d'analgésiques dérivés de la morphine à des doses usuelles sont connus :
1 - nausée, vomissements
2 - constipation
3 - dépression respiratoire
4 - accoutumance physique et/ou psychique au cours de traitements prolongés
5 - syndrome de manque à l'arrêt du traitement comportant : mydriase, contractions musculaires, céphalées, sudation, vomissements, diarrhées, tachycardie, polypnée, hyperthermie, hypertension ...

L'effet de synergie analgésique, obtenu grâce à l'association se révèle donc particulièrement utile pour amoindrir la dépendance physique et psychique ainsi que l'accoutumance se développant à la suit d'administrations répétées d'analgésiques de type morphinique.

On peut donc parler de l'association forskoline avec un analgésique morphinique comme d'un "économiseur de morphine".

L'invention concerne donc notamment l'association caractérisée en ce que l'analgésique central qu'elle renferme est la morphine.

La potentialisation de l'effet de l'analgésique central est illustrée par des études pharmacologiques chez la souris, rapportées plus loin.

D'après le résultat de ces études, l'association permet par exemple de réduire d'environ deux à cinq fois la dose de morphine employée.

L'invention s'étend aux compositions pharmaceutiques renfermant l'association, objet de l'invention.

Les compositions pharmaceutiques peuvent être utilisées dans le traitement des douleurs intenses, en particulier rebelles aux antalgiques périphériques, par exemple, au cours des processus néoplasiques, dans le traitement des pancréatites, coliques néphrétiques ou biliaires, dans le traitement des douleurs post opératoires et post traumatiques.

Les compositions pharmaceutiques peuvent être administrées par voie buccale, parentérale ou rectale.

Ces compositions pharmaceutiques peuvent être, par exemple, solides ou liquides et se présenter sous les formes pharmaceutiques couramment utilisées en médecine humaine comme, par exemple, les comprimés, simples ou dragéifiés, les gélules, les capsules, les granulés, les préparations injectables, les suppositoires ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, les véhicules aqueux ou non, les corps gras d'origine animale ou végétale, les dérivés paraffiniques, les glycols, les divers agents mouillants, dispersants ou émulsifiants, les conservateurs.

L'invention s'étend à l'utilisation d'un analgésique central et de la forskoline pour la préparation des compositions pharmaceutiques telles que définies ci-dessus.

Les proportions relatives des constituants de l'association, objet de l'invention, sont variables.

Elles peuvent être, par exemple, de 1 partie en poids de forskoline pour 0,01 à 20 parties en poids d'analgésique central et de préférence pour 0,1 à 20 parties d'analgésique central.

Les proportions dépendent fortement de la puissance d'activité de l'analgésique central utilisé, selon que l'analgésique choisi est la morphine ou un analgésique plus actif que la morphine (péthidine -fentanyl - dextromoramide) ou moins actif que la morphine (pentazocine).

La posologie varie en fonction de l'affection traitée, du sujet en cause et de la voie d'administration. On peut donner par exemple, pour 1 partie en poids d'analgésique central, 5 à 20 parties de forskoline administrée par voie orale et 0,2 à 5 parties de forskoline administrée par voie sous-cutanée.

Lorsque les deux constituants s de l'association ion selon l'invention, sont administrés par voie parentérale ou orale, ils peuvent être administrés en mélange, préparé extemporanément ou non. Ils peuvent aussi être administrés successivement, à des intervalles pouvant aller de quelques secondes à une ou plusieurs heures jusqu'à 15 heures.

Lorsque les deux constituants de l'association sont administrés par voie parentérale, ils sont administrés de préférence, simultanément.

Les deux constituants de l'association peuvent être administrés par des voies d'administration différentes et peuvent donc se présenter sous la forme d'un kit à 2 composants contenant d'une part la forskoline sous une forme pharmaceutique destinée à la voie orale et d'autre part, l'analgésique sous une forme pharmaceutique destinée à la voie parentérale.

Exemple 1 : Compositions pharmaceutiques.

1) On a préparé un soluté injectable répondant à la formule :
- chlorhydrate de morphine............5 mg
- forskoline............10 mg
- solvant stérile............2 ml
2) On a préparé extemporanément un soluté injectable répondant à la formule :
- soluté injectable de chlorhydrate de morphine à 0,25%.. 2 m
- forskoline............5 mg
On prépare le soluté injectable en introduisant au moment de l'emploi le soluté injectable de chlorhydrate de morphine à 0,25 % sur la forskoline et en agitant.
3) kit pharmaceutique, constitué :
- d'un comprimé répondant à la formule :
Forskoline............50 mg
Excipient q.s. pour un comprimé terminé à............350 mg
(lactose, amidon, stéarate de magnésium, talc).
- d'un soluté injectable de chlorhydrate de morphine à 0,25 %............2 ml.

Exemple 2 : Etude pharmacologique.

Potentialisation de l'activité analgésique de la morphine sur le test de la plaque chaude chez la souris.

Des souris femelles pesant de 20 à 22 g sont placés, une par une, sur une plaque de cuivre maintenue à 56¤C : la réaction à la douleur se manifeste par le léchage d'une patte ou des deux pattes antérieures ; le temps de cette réaction est noté et l'on ne retient que les souris réagissant en moins de 10 secondes.

Les souris sont réparties en groupes homogènes, un groupe ne recevant que le véhicule des produits administrés.

La forskoline est administrée à une dose inactive par elle-même de 5 mg/kg par voie sous-cutanée ou de 50 mg/kg par voie orale, simultanément avec une injection de chlorhydrate de morphine dans le premier cas et 5 heures 30 minutes avant l'injection de chlorhydrate de morphine dans le second cas.

La réactivité des souris à la douleur est notée 30 minutes après le traitement.

3

AUGMENTATION DU TEMPS DE REACTION EN % 30 MINUTES APRES
L'ADMINISTRATION DE MORPHINE.

```
-Morphine 3 mg/kg  s.c.                          102
-Forskoline 5 mg/kg s.c. + ⎫
 Morphine   3 mg/kg s.c.   ⎬                      209
                          ⎭

-Morphine   3 mg/kg s.c.                          86
-Forskoline 50 mg/kg v.o + ⎫
 Morphine   3 mg/kg s.c.   ⎬                      170
                          ⎭
```

**Revendications**

1.  Association renfermant, à titre de principes actifs :
    - a) un analgésique central choisi parmi la morphine, la péthidine, le fentanyl, la pentazocine et le dextromoramide, et
    - b) la forskoline, pour une utilisation simultanée, séparée ou étalée dans le temps, pour une méthode de traitement analgésique du corps humain ou animal.

2.  Association selon la revendication 1, caractérisée en ce que l'analgésique central qu'elle renferme est la morphine.

3.  Les compositions pharmaceutiques, contenant à titre de principes actifs, l'une au moins des associations telles que définies à l'une quelconque des revendications 1 ou 2.

**Claims**

1.  Combination containing, as active ingredients:
    - a) a central analgesic chosen from morphine, pethidine, fentanyl, pentazocine and dextromoramide, and
    - b) forskolin, for use which is simultaneous, separate or spread out over time, for an analgesic treatment method for humans or animals.

2.  Combination according to claim 1, characterized in that the central analgesic that it contains is morphine.

3.  The pharmaceutical compositions, containing as as active ingredients, at least one of the combinations defined in any one of claims 1 or 2.

**Patentansprüche**

1.  Assoziation enthaltend als Wirkstoff
    a) ein zentrales Analgetikum, ausgewählt unter Morphin, Pethidin, Fentanyl, Pentazocin und Dextromoramid, und
    b) Forskolin für eine gleichzeitige, getrennte oder zeitlich verschobene Anwendung bei einem Verfahren zur analgetischen Behandlung des menschlichen oder tierischen Körpers.

2.  Assoziation gemäß Anspruch 1, dadurch gekennzeichnet, daß das zentrale Analgetikum, welches sie enthält, das Morphin ist.

3.  Pharmazeutische Zusammensetzungen, enthaltend als Wirkstoff zumindest eine der Assoziationen wie in einem der Ansprüche 1 oder 2 definiert.